# FASCICULE DE BREVET EUROPEEN

(11) **EP 3 080 144 B1**
(45) Date de publication et mention de la délivrance du brevet: **14.07.2021**
(21) Numéro de dépôt: 14821808.4
(22) Date de dépôt: 08.12.2014
(51) Int. Cl.: C07K 14/195, C07K 14/33, C12N 9/96

(54) **STABILISATION DE LA GLUTAMATE DÉSHYDROGÉNASE EN SOLUTION AQUEUSE**
STABILISIERUNG VON GLUTAMAT-DEHYDROGENASE IN EINER WÄSSRIGEN LÖSUNG
STABILIZATION OF GLUTAMATE DEHYDROGENASE IN AN AQUEOUS SOLUTION

(30) Priorité: 10.12.2013 FR 1362354
(43) Date de publication de la demande: 19.10.2016
(73) Titulaire: Biomérieux, 69280 Marcy l'Étoile (FR)
(72) Inventeur: BETTSWORTH, Florence, F-69380 Dommartin (FR); MARTINEZ, Jérôme, F-69003 Lyon (FR)
(74) Mandataire: El Mardeny, Galal
(86) Numéro de dépôt international: PCT/FR2014/053213
(87) Numéro de publication internationale: WO 2015/086973

(56) Documents cités:
- WO-A1-98/45706
- WO-A1-2007/003936
- WO-A2-2008/084237
- WO-A2-2009/006301
- KATHRYN S. LILLEY ET AL: "The essentail active-site lysines of clostridial glutamate dehydrogenase. A study with pyridoxal-5'-phophate", EUROPEAN JOURNAL OF BIOCHEMISTRY, vol. 207, no. 2, 1 juillet 1992 (1992-07-01), pages 533-540, XP055128606, ISSN: 0014-2956, DOI: 10.1111/j.1432-1033.1992.tb17079.x
- MICHAEL R. MAURIZI ET AL: "Degradation of -Glutamate Dehydrogenase from Escherichia coli: Allosteric Regulation of Enzyme Stability", ARCHIVES OF BIOCHEMISTRY AND BIOPHYSICS, vol. 397, no. 2, 1 janvier 2002 (2002-01-01), pages 206-216, XP55128604, ISSN: 0003-9861, DOI: 10.1006/abbi.2001.2703
- MICHAEL R. MAURIZI ET AL: "Degradation of -Glutamate Dehydrogenase from Escherichia coli: Allosteric Regulation of Enzyme Stability", ARCHIVES OF BIOCHEMISTRY AND BIOPHYSICS, vol. 397, no. 2, 1 janvier 2002 (2002-01-01), pages 206-216, XP055128604, ISSN: 0003-9861, DOI: 10.1006/abbi.2001.2703
- None

## Description

La présente invention concerne le domaine de la détection *in vitro* de protéines de bactéries dans des échantillons biologiques susceptibles de contenir ces protéines. En particulier, l'invention concerne la stabilisation de la glutamate déshydrogénase d'une bactérie du genre *Clostridium* afin que cette protéine conserve ses propriétés antigéniques lorsqu'elle est en solution aqueuse.

Les bactéries du genre *Clostridium* sont des bactéries anaérobies, sporulées, à Gram positif qui sont incapables de réduire les sulfates en sulfites. Certaines espèces sont très pathogènes, telles que *Clostridium difficile, Clostridium botulinum* et *Clostridium perfringens* pour les plus connues.

La bactérie *Clostridium difficile* est le principal agent responsable de la diarrhée suite à l'administration d'antibiotiques. Elle est redoutable en raison de son potentiel de contagion très élevé. Bien qu'environ 5 % de la population soient porteurs asymptomatiques de la bactérie, ses manifestations pathologiques sont étroitement reliées à un séjour à l'hôpital. Cette bactérie se développe dans une flore intestinale affaiblie par l'antibiothérapie et peut secréter deux toxines, A et B. Seules les souches produisant des toxines sont pathogènes. La toxine A, une entérotoxine, provoque l'altération de la perméabilité de l'épithélium intestinal ; la toxine B, une cytotoxine, s'attaque directement aux cellules de l'épithélium. L'effet combiné des deux toxines est la diminution du temps de transit intestinal et de l'absorption intestinale, ce qui résulte en une diarrhée. Plus rarement, la bactérie *Clostridium difficile* peut provoquer une inflammation sévère du colon (colite pseudomembraneuse).

La bactérie *Clostridium botulinum* quant à elle est responsable du botulisme. Elle produit des spores qui représentent la forme de résistance de la bactérie. Ces spores peuvent résister à de faibles traitements thermiques, tels que la pasteurisation, ce qui peut poser des problèmes en sécurité alimentaire, puis donner une cellule bactérienne métaboliquement active, capable de se multiplier. Cette bactérie sécrète une des toxines les plus puissantes du monde vivant, la toxine botulique. Active par ingestion, cette toxine diffuse ensuite dans l'organisme et agit en bloquant la transmission neuromusculaire : elle inhibe les neurones moteurs de la contraction musculaire. Cette infection peut entrainer la mort par paralysie des muscles respiratoires si aucun traitement n'est mis en place.

La bactérie *Clostridium perfringens* est une bactérie qui se développe dans les plaies, parfois de façon très profonde. Cette bactérie va produire des nécrotoxines provoquant ainsi l'entérite nécrosante. La toxine majeure la plus fréquente est la toxine alpha, essentiellement produite par *Clostridium perfringens* type A. Cette toxine est impliquée dans de très nombreux cas de gangrène chez l'homme et les animaux. Seule ou en association avec d'autres toxines, elle cause également des mortalités brutales chez les porcs et les ruminants.

La détection de la présence de ces bactéries et de la sécrétion de leur toxine est donc un problème majeur de santé publique qui nécessite pour les laboratoires d'avoir des tests de détection fiables, tant au niveau de la sensibilité/spécificité, qu'au niveau de la reproductibilité des résultats obtenus avec ces tests. Pour ce faire, les laboratoires ont besoin notamment de tests incluant des réactifs qui ne se dégradent pas dans le temps et donc restent stables.

La détection de la présence de bactéries dans des échantillons peut être mise en œuvre par différentes techniques telles que l'utilisation de milieux de culture ou la technique des immunodosages, lesquels sont largement connues de l'homme du métier. La technique des immunodosages est une technique consistant dans les grandes lignes à détecter la présence de protéines en utilisant des partenaires de liaison de ces protéines. Dans le cadre de la détection des bactéries du genre *Clostridium,* l'une des protéines détectables, représentatives de la présence de cette bactérie, est la glutamate déshydrogénase (GDH). D'autres protéines détectables sont les toxines sécrétées lorsque les bactéries sont toxinogènes. La détection ou quantification par immunodosage de la GDH est une technique qui permet d'avoir une sensibilité diagnostique plus grande que la détection ou quantification par immunodosage des toxines. Elle est utilisée en tant que moyen de criblage sur les populations à risque. En cas de positivité, une recherche des toxines est alors recommandée car cette technique présente quant à elle une plus grande spécificité.

Plusieurs sociétés de diagnostic proposent des trousses pour la détection de la GDH chez *Clostridium difficile.* On peut citer par exemple la trousse VIDAS® GDH de la Demanderesse.

Outre les partenaires de liaison, la technique d'immunodosage nécessite également l'utilisation de réactifs pour la calibration et/ou le contrôle du test. Ainsi, dans le cadre d'un immunodosage de la GDH, de tels réactifs comprennent la GDH en tant que telle, laquelle doit conserver ses propriétés antigéniques aussi longtemps que nécessaire au regard de la durée de vie de la trousse de diagnostic dans laquelle elle est contenue.

Les propriétés d'une protéine peuvent être perturbées par toute modification de structure, tant d'un point de vue chimique que physique. Les modifications chimiques d'une protéine sont basées sur des changements au niveau des liaisons covalentes, dus par exemple à des réactions d'oxydation, hydrolyse, etc., tandis que les modifications physiques, également appelées dénaturation, entrainent une désorganisation de la structure tertiaire ou conformation tridimensionnelle de la protéine, sans rupture des liaisons covalentes. La dénaturation des protéines peut être induite par de nombreux facteurs, chimiques ou physiques, tels que, entre autres, la température, la modification du pH ou un agent chimique. De telles modifications ont comme conséquence une perturbation de l'activité propre de la protéine. Ainsi, dans le cadre d'une enzyme comme la GDH, de telles modifications peuvent altérer son activité enzymatique, ce que les différents auteurs ont essayé de pallier.

Ainsi, par exemple, les auteurs de la demande de brevet WO2007/003936 ont décrit la stabilisation de l'activité enzymatique de différentes protéines en utilisant un ou plusieurs composés stabilisants ayant les caractéristiques suivantes :
- Ils ont des groupes ionisables capables d'échange de protons avec la protéine à stabiliser et avec les produits ionisés de la solution aqueuse,
- Les groupes ionisables incluent des premiers groupements qui sont chargés positivement lorsqu'ils sont protonés et non chargés quand ils sont déprotonés, et des seconds groupements qui sont non chargés lorsqu'ils sont protonés et négativement chargés quand ils sont déprotonés, et
- Le pH de la composition est maintenue dans une gamme de +/- 0,5 unités pH du pH auquel la composition possède sa stabilité maximale par rapport au pH.
Ce document décrit que l'activité enzymatique de la GDH bovine est conservée par ajout de lysine comme composé stabilisant, alors que l'ajout de citrate ne permet pas à la GDH de conserver cette activité enzymatique.

Garcia-Galan C. *et al.,* 2013, ont quant à eux décrit que la GDH de *Escherichia coli* pouvait être stabilisée pour qu'elle maintienne son activité enzymatique en revêtant la surface de la GDH par de la polyéthylèneimine en présence de Lithium⁺.

Toutefois, aucun auteur ne s'est intéressé à rechercher comment stabiliser la GDH pour qu'elle conserve ses propriétés antigéniques, alors que c'est un problème rencontré lorsque cette protéine est utilisée en solution aqueuse, notamment de façon très diluée, par exemple à une concentration de l'ordre de quelques ng/mL.

En effet, la GDH est habituellement conservée sous forme lyophilisée car il est connu qu'elle ne conserve pas ses propriétés antigéniques lorsqu'elle est mise en solution aqueuse. Aussi, lorsque la GDH doit être mise en solution aqueuse, par exemple dans le cadre d'un immunodosage de la GDH, le laborantin reprend la GDH lyophilisée dans un solvant aqueux, prépare des aliquotes qu'il doit ensuite congeler à -20°C. La durée de péremption de la solution aqueuse est alors assez courte, en moyenne de 2 mois conservée entre 2 et 8°C. De plus, le fait de reprendre la GDH dans un tampon aqueux a pour inconvénients d'entraîner non seulement des manipulations supplémentaires, mais également des risques supplémentaires d'erreur à la reprise. Enfin, cela nécessite également la présence d'un congélateur.

La Demanderesse a trouvé contre toute attente qu'il était possible de stabiliser la GDH en solution aqueuse afin que sa structure tridimensionnelle soit conservée au moins en partie de sorte qu'elle garde ses propriétés antigéniques. Une telle stabilisation est mise en œuvre par addition, en tant que composé de stabilisation, d'un acide carboxylique ayant une chaîne carbonée d'au moins 3 atomes de carbone et comportant au moins 2 groupements -COOH ou d'un de ses sels. Grâce à l'ajout de ce composé, la solution aqueuse comprenant la GDH peut être conservée entre 2 et 8°C, et ce pendant de nombreux mois.

Ainsi, un premier objet de l'invention concerne un procédé de détection de la présence d'une bactérie du genre *Clostridium* dans un échantillon biologique susceptible de contenir une telle bactérie, caractérisé en ce qu'il comprend les étapes de :
(i) mettre un œuvre un procédé de détection de la présence d'une bactérie du genre *Clostridium* par détection ou quantification de la présence de glutamate déshydrogénase dans ledit échantillon en utilisant une composition aqueuse comprenant la glutamate déshydrogénase d'une bactérie du genre *Clostridium* et un composé de stabilisation qui est un acide carboxylique ayant une chaîne carbonée d'au moins 3 atomes de carbone et comportant au moins 2 groupements -COOH ou un de ses sels, et
(ii) un résultat positif à l'étape (i) permet de conclure à la présence de la bactérie.

Un autre objet de l'invention concerne un procédé de détection de la présence d'une bactérie toxinogène du genre *Clostridium* produisant au moins une toxine, dans un échantillon biologique susceptible de contenir une telle bactérie et une telle au moins une toxine.

La Demanderesse a donc montré contre toute attente que l'utilisation de composés particuliers permettait de stabiliser la GDH des bactéries du genre *Clostridium* pour maintenir ses propriétés antigéniques lorsque la GDH se trouve en solution aqueuse, notamment dans des concentrations de l'ordre de quelques ng/mL, par exemple de 0,75 à 10 ng/mL, de 2 à 10 ng/mL ou encore de 3 à 6 ng/mL, ce qui est particulièrement important dans le cadre des tests pour la détection de ces bactéries par immunodosage.

Par maintien des propriétés antigéniques de la GDH, on entend que la GDH conserve sa propriété de liaison aux partenaires de liaison mis en œuvre dans le cadre de l'immunodosage car sa structure est conservée, au moins au niveau du déterminant antigénique impliqué dans la fixation du partenaire de liaison.

Bien entendu, le préfixe « immuno » dans le terme « immunodosage », par exemple, n'est pas à considérer dans la présente demande comme indiquant strictement que le partenaire de liaison est nécessairement un partenaire d'origine immunologique, tel qu'un anticorps ou un fragment d'anticorps. En effet, comme cela est bien connu de l'homme du métier, ce terme est plus largement utilisé pour désigner des tests et procédés dans lesquels le partenaire de liaison n'est pas un partenaire d'origine/de nature immunologique mais consiste, par exemple, en un récepteur de l'analyte que l'on souhaite détecter et/ou quantifier. La condition étant que le partenaire de liaison concerné soit capable de se lier à l'analyte recherché, de préférence de manière spécifique. Ainsi, il est connu de parler du dosage ELISA pour des dosages qui utilisent des partenaires de liaison non immunologiques *stricto sensu,* appelés plus largement en anglais « ligand binding assay », que l'on pourrait traduire en langue française par « dosage utilisant la liaison à un ligand », alors que le terme « immuno » est inclus dans l'intitulé *in extenso* correspondant à l'acronyme ELISA. Dans un souci de clarté et d'uniformité, le terme « immuno » est employé dans la présente demande pour désigner toute analyse biologique utilisant au moins un partenaire de liaison adapté pour se lier à l'analyte recherché et détecter et/ou quantifier ce dernier, de préférence de manière spécifique, même quand ledit partenaire de liaison n'est pas de nature ou d'origine immunologique au sens strict.

Par partenaire de liaison à la GDH, on entend toute molécule capable de se lier à la GDH. A titre d'exemple de partenaire de liaison à la GDH, on peut citer les anticorps, les fragments d'anticorps, les nanofitines, les récepteurs à la GDH, les aptamères, les DARPins ou toute autre molécule qui est connue pour avoir une interaction avec la GDH.

Les anticorps partenaires de liaison sont par exemple soit des anticorps polyclonaux, soit des anticorps monoclonaux.

Les anticorps polyclonaux peuvent être obtenus par immunisation d'un animal avec, comme immunogène, la GDH cible, suivie de la récupération des anticorps recherchés sous forme purifiée, par prélèvement du sérum dudit animal, et séparation desdits anticorps des autres constituants du sérum, par exemple par chromatographie d'affinité sur une colonne sur laquelle est fixé un antigène spécifiquement reconnu par les anticorps, notamment l'immunogène, ou à l'aide de d'une protéine A ou G.

Les anticorps monoclonaux peuvent être obtenus par la technique des hybridomes largement connue de l'homme du métier. Les anticorps monoclonaux peuvent être également des anticorps recombinants obtenus par génie génétique, par des techniques bien connues de l'homme du métier.

A titre d'exemple de fragments d'anticorps, on peut citer les fragments Fab, Fab', F(ab')2 ainsi que les scFv (Single chain variable fragment), dsFv (Double-stranded variable fragment). Ces fragments fonctionnels peuvent notamment être obtenus par génie génétique.

Les nanofitines (nom commercial) sont de petites protéines qui, comme les anticorps, sont capables de se lier à une cible biologique permettant ainsi de la détecter, de la capturer ou tout simplement de la cibler au sein d'un organisme.

Les aptamères sont des oligonucléotides, généralement ARN ou ADN, identifiés dans des banques contenant jusqu'à 10¹⁵ séquences différentes, par une méthode combinatoire de sélection in vitro appelée SELEX pour « Systematic Evolution of Ligands by Exponentiel Enrichment » (Ellington AD et Szostak JW., 1990). La plupart des aptamères sont composés d'ARN, en raison de la capacité de l'ARN à adopter des structures variées et complexes, ce qui permet de créer à sa surface des cavités de géométries variées, permettant de fixer des ligands divers. Il s'agit d'outils biochimiques d'intérêt qui peuvent être utilisés dans des applications biotechnologiques, diagnostiques ou thérapeutiques. Leur sélectivité et leurs propriétés de fixation de ligands sont comparables à celle des anticorps.

Les « DARPins » pour Designed Ankyrin Repeat ProteINS (Boersma YL et Plütckthun A, 2011) sont une autre classe de protéines permettant de mimer les anticorps et de pouvoir se fixer avec une affinité et une sélectivité élevées sur des protéines cibles. Ils dérivent de la famille des protéines ankyrines qui sont des protéines adaptatrices permettant de fixer les protéines de membrane intégrales au réseau spectrine/actine qui constitue « la colonne vertébrale » de la membrane plasmatique cellulaire. La structure des ankyrines est basée sur la répétition d'un motif d'environ 33 acides aminés et il en est de même des DARPins. Chaque motif a une structure secondaire de type hélice-coude-hélice (« helix-turn-helix »). Les DARPins contiennent au moins trois, de préférence quatre à cinq motifs répétés et sont obtenus par screening de banques combinatoires.

Les partenaires de liaison utilisés peuvent être spécifiques ou non de la GDH. Ils sont dits spécifiques quand ils sont capables de se lier de façon exclusive ou quasi exclusive à la GDH. Ils sont dits non spécifiques lorsque la sélectivité de liaison à la GDH est plus faible et qu'ils sont alors capables de se lier à d'autres ligands, tels que d'autres protéines ou anticorps. Selon un mode de réalisation préféré, on préfère les partenaires de liaison spécifiques.

La glutamate déshydrogénase qu'il convient de stabiliser est toute glutamate déshydrogénase de *Clostridium* dont on veut détecter la présence, par exemple celle de *Clostridium difficile,* de *Clostridium botulinum* ou de *Clostridium perfringens.* Elle inclut tous les variants possibles. De telles protéines sont connues et leurs séquences sont décrites par exemple dans la base de données Uniprot (www.uniprot.org).

Ainsi, la GDH de *Clostridium difficile* (N° accession Uniprot P27346) est une protéine de 421 acides aminés dont la séquence de référence en acides aminés est la SEQ ID N°1 suivante :

Et dont les variants sont :

**Tableau 1**

| N° accession Uniprot des variants | Souche |
|---|---|
| Q18CS0 | *Clostridium difficile* (strain 630) |
| C9XIV3 | *Clostridium difficile* (strain CD196) |
| C9YHY9 | *Clostridium difficile* (strain R20291) |
| G6B2V9 | *Clostridium difficile* 002-P50-2011 |
| G6BHV4 | *Clostridium difficile* 050-P50-2011 |
| D5S4M2 | *Clostridium difficile* NAP07 |
| G6BQY2 | *Clostridium difficile* 70-100-2010 |
| D5Q9B1 | *Clostridium difficile* NAP08 |

La GDH de *Clostridium perfringens* est une protéine n'ayant pas encore de séquence de référence dans la base Uniprot. La première protéine donnée dans la base Uniprot (N° accession Uniprot Q8XK85) est la protéine de la souche 13 de type A, de 448 acides aminés, dont la séquence en acides aminés est la SEQ ID N°2 suivante :

Et dont les variants sont :

**Tableau 2**

| N° accession Uniprot des variants | Souche |
|---|---|
| Q8XK85 | *Clostridium perfringens* (strain 13 / Type A) |
| Q0SST9 | *Clostridium perfringens* (strain SM101 / Type A) |
| Q0TQ84 | *Clostridium perfringens* (strain ATCC 13124 / NCTC 8237 / Type A) |
| B1R556 | *Clostridium perfringens B* str. ATCC 3626 |
| B1BJJ0 | *Clostridium perfringens C* str. JGS1495 |
| B1BWI0 | *Clostridium perfringens E* str. JGS1987 |
| B1RGN1 | *Clostridium perfringens CPE* str. F4969 |
| B1V119 | *Clostridium perfringens D* str. JGS1721 |
| B1RPY4 | *Clostridium perfringens NCTC* 8239 |
| H7CWP7 | *Clostridium perfringens* F262 |
| H1CRA8 | *Clostridium perfringens* WAL-14572 |

La GDH de *Clostridium botulinum* est une protéine n'ayant pas encore de séquence de référence dans la base Uniprot. La première protéine donnée dans la base Uniprot (N° accession Uniprot A5I2T3) est la protéine de la souche Hall de type A (ATCC 3502, NCTC 13319), de 421 acides aminés, dont la séquence en acides aminés est la SEQ ID N°3 suivante :

Et dont les variants, de 421, 447 ou 450 acides aminés selon les souches, sont :

**Tableau 3**

| N° accession Uniprot des variants | Souche |
|---|---|
| B1IM79 | *Clostridium botulinum* (strain Okra / Type B1)421 |
| B1KSB4 | *Clostridium botulinum* (strain Loch Maree / Type A3) |
| A7FUM1 | *Clostridium botulinum* (strain ATCC 19397 / Type A) |
| E8ZRR3 | *Clostridium botulinum* (strain H04402 065 / Type A5) |
| B2TLD1 | *Clostridium botulinum* (strain Eklund 17B / Type B) |
| C1FNV0 | *Clostridium botulinum* (strain Kyoto / Type A2) |
| B2V1W6 | *Clostridium botulinum* (strain Alaska E43 / Type E3) |
| C3KX46 | *Clostridium botulinum* (strain 657 / Type Ba4) |
| D5VZM8 | *Clostridium botulinum* (strain 230613 / Type F) |
| F4A4K5 | *Clostridium botulinum* BKT015925 |
| A7GE56 | *Clostridium botulinum* (strain Langeland / NCTC 10281 / Type F) |
| B1B9U1 | *Clostridium botulinum* Cstr. Eklund |
| C5VRL1 | *Clostridium botulinum* D str. 1873 |
| B1QDG7 | *Clostridium botulinum* NCTC 2916 |
| B1QQR1 | *Clostridium botulinum* Bf |
| M1ZQM8 | *Clostridium botulinum* CFSAN001627 |
| L1LK36 | *Clostridium botulinum* CFSAN001628 |
| C5UPY2 | *Clostridium botulinum* E1 str. 'BoNT E Beluga' |

Selon un mode de réalisation particulier, la glutamate déshydrogénase est une enzyme de la bactérie de l'espèce *Clostridium difficile.*

La glutamate déshydrogénase mise en solution aqueuse est soit d'origine naturelle, soit d'origine recombinante. La glutamate déshydrogénase naturelle ou encore appelée native peut être obtenue après culture de la bactérie *Clostridium* et purification de la protéine à partir du lysat bactérien. La glutamate déshydrogénase recombinante peut être obtenue par génie génétique, par des techniques bien connues de l'homme du métier. Une telle obtention est décrite par exemple par Anderson BM *et al.,* 1993. La glutamate déshydrogénase recombinante peut être obtenue auprès de Sociétés telles que Holzel Diagnostika GmbH (Allemagne).

Par composition ou solution aqueuse, on entend une solution liquide et limpide obtenue par dissolution complète d'un ou plusieurs composés et dont le solvant majoritaire est l'eau, représentant au moins 50% en volume, en général au moins 60%, 70%, 80%, 90%, par rapport au volume total de la solution.

Dans le cadre de la présente invention, la composition ou solution aqueuse est obtenue en diluant la GDH dans un solvant comprenant majoritairement de l'eau et un composé de stabilisation tel que défini ci-après.Le composé de stabilisation à ajouter dans la solution aqueuse contenant la GDH à stabiliser est un acide carboxylique ayant une chaîne carbonée d'au moins 3 atomes de carbone et comportant au moins 2 groupements -COOH, ou un de ses sels.

Par acide carboxylique ayant une chaîne carbonée d'au moins 3 atomes de carbone et comportant au moins 2 groupements -COOH, on entend une molécule constituée de :
- une chaîne carbonée, linéaire ou ramifiée, contigüe (c'est-à-dire sans interruption dans la chaîne carbonée) ou interrompue par au moins un autre atome différent de l'atome de carbone, par exemple un atome d'azote ou d'oxygène,
- au moins 2 groupements -COOH en bout de chaîne et
- au moins un groupement « CX », soit en bout de chaîne (il s'écrit alors -CX), soit en milieu de chaîne (il s'écrit alors -CX-), X étant différent de C.

Par exemple, les groupements -CX- sont choisis indépendamment parmi -CH₂-, =CH-, -C(H)OH-, -C(H)NH₂- et -C(O)-. Les groupements -CX sont quant à eux choisis indépendamment parmi -CH₃, -COOH (s'il y a, dans la molécule, plus de 2 groupements -COOH) et -C(O)NH₂.

Ainsi, par exemple, le composé de stabilisation peut être l'acide succinique, de formule OH(O)C-(CH₂)₂-C(O)OH, qui est une molécule ayant une chaîne carbonée linéaire contigüe de 4 atomes de carbone, 2 groupements -COOH en bout de chaîne et 2 groupements -CH₂-.

Un autre exemple consiste en l'acide fumarique de formule OH(O)C-(CH=CH)-C(O)OH, qui est une molécule ayant une chaîne carbonée linéaire contigüe de 4 atomes de carbone, 2 groupements -COOH en bout de chaîne et 2 groupements -CH-.

Encore un autre exemple consiste en l'acide N-(2-acétamido)iminodiacétique, de formule H₂NC(O)-CH₂-N(CH₂-COOH)₂, qui est une molécule ayant une chaîne carbonée ramifiée de 6 atomes de carbone, interrompue par un atome d'azote, constituée de 2 groupements -COOH en bout de chaîne, de 3 groupements -CH₂- et d'1 groupement -C(O)NH₂.

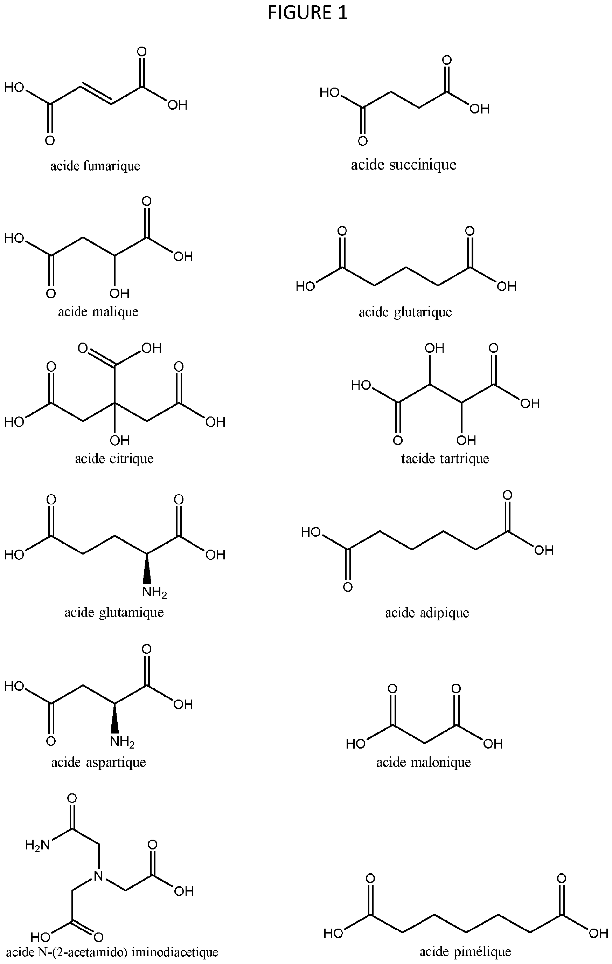

Selon un mode de réalisation particulier, le composé de stabilisation est choisi parmi : l'acide fumarique, l'acide succinique, l'acide malique, l'acide glutarique, l'acide citrique, l'acide tartrique, l'acide N-(2-acétamido)iminodiacétique, l'acide glutamique, l'acide adipique, l'acide aspartique, l'acide pimélique, l'acide malonique et leurs sels, dont les formules sont données sur la Figure 1.

Par sel d'acide carboxylique, on entend un sel d'un cation monovalent. A titre de cation monovalent, on peut citer les ions ammonium (NH4⁺), argent (Ag⁺), diaminoargent (Ag(NH₃)₂⁺), césium (Cs⁺), cuivre(I) (Cu⁺), mercure (Hg⁺), le méthanium (CH₅⁺), le méthylium (CH3⁺), le nitrosium (NO₂⁺) et les ions des métaux alcalins sodium (Na⁺), potassium (K⁺) et lithium (Li⁺).

Lorsque le composé de stabilisation est sous forme de sel, au moins un proton H⁺ des groupements -COOH est remplacé par un cation monovalent décrit ci-dessus.

Selon un mode de réalisation préféré, le composé de stabilisation est choisi parmi l'acide succinique, l'acide fumarique et leurs sels, en particulier de métaux alcalins, tels que définis ci-dessus.

La chaîne carbonée peut comprendre une ou plusieurs des caractéristiques suivantes :
- elle peut comprendre au moins 3 atomes de carbone à au plus 10 atomes, de préférence au plus 8, de préférence au plus 7, de préférence au plus 6 atomes,
- elle intercale un atome d'azote ou elle est contigüe et n'est constituée que d'atomes de carbone,
- les groupements « CX » sont choisis indépendamment parmi -CH₂-, =CH-, -CH₃, -COOH, -C(H)OH-, -C(O)NH₂, -C(H)NH₂-, -C(O) -, et
- elle peut comprendre ou non une ou plusieurs doubles liaisons et l'acide peut alors se trouver sous forme d'isomère E ou Z.

En particulier, l'acide carboxylique peut présenter une ou plusieurs des caractéristiques suivantes :
- une chaîne carbonée de 4, 5 ou 6 atomes de carbone,
- une chaîne carbonée de 4 atomes de carbone ayant au moins 2 groupements -COOH et des groupements -CX- choisis indépendamment parmi =CH-, -CH₂- et -C(H)OH-,
- une chaîne carbonée de 5 atomes de carbone ayant au moins 2 groupements -COOH et des groupements -CX- choisis parmi -CH₂- et -C(H)NH₂,
- une chaîne carbonée de 6 atomes de carbone ayant au moins 2 groupements -COOH et des groupements « -CX- » choisis parmi -CH2-, -C(H)OH-(-CX-) et -C(O)NH₂ (-CX),
- une double liaison et est un isomère E,
- 2 groupements -COOH.

La quantité de GDH présente dans la solution aqueuse dépend de l'utilisation finale de la composition aqueuse qui la contient. Dans le cadre d'un immunodosage classique, la GDH peut être présente à raison de 0,75 à 10 ng/mL, ou de 2 à 10 ng/mL, de préférence de 3 à 6 ng/mL. Dans le cadre d'un immunodosage dit ultrasensible, la GDH sera présente en une quantité bien inférieure, par exemple inférieure au pg/mL, voire de l'ordre du fg/mL.

La quantité de composé de stabilisation à ajouter dans la solution aqueuse contenant la GDH est en large excès par rapport à la quantité de GDH. Elle dépend du fait que le composé de stabilisation est utilisé uniquement à titre de composé de stabilisation, une autre molécule étant alors ajoutée à titre de tampon, ou bien s'il est utilisé à la fois à titre de composé de stabilisation et de tampon. Ainsi, par exemple, lorsque le composé de stabilisation est uniquement utilisé à titre de composé de stabilisation, on ajoute de 20 à 100 molécules de composé de stabilisation par monomère de GDH, de préférence de 30 à 80 molécules de composé de stabilisation par monomère de GDH, de préférence encore de 40 à 60 molécules de composé de stabilisation par monomère de GDH. Dans ce cas, le composé ajouté à titre de tampon, est tout composé connu de l'homme du métier ayant un pH compris entre 4,5 et 7, de préférence entre 5,5 et 6,5, un pH de 5,8 étant préféré. A titre d'exemple de composé ajouté à titre de tampon, on peut citer le phosphate et l'acétate. Lorsque le composé de stabilisation est utilisé à la fois à titre de composé de stabilisation et de tampon, on ajoute de 10⁸ à 10¹⁰ molécules de composé de stabilisation par monomère de GDH, de préférence de 1×10⁹ à 5×10⁹ molécules de composé de stabilisation par monomère de GDH, de préférence encore de 1×10⁹ à 2×10⁹ molécules de composé de stabilisation par monomère de GDH.

D'autres composés peuvent également être ajoutés à la composition aqueuse dans le procédé de l'invention. Ainsi, par exemple, un polyol peut être ajouté. L'ajout de polyol permet de favoriser la stabilité thermique des protéines (résistance à la dénaturation thermique) et aussi de prévenir l'agrégation. De manière générale les polyols ont un effet co-solvant et contribuent au maintien de la conformation native des protéines en solution aqueuse.

A titre d'exemples de polyol, on peut citer les polyols monosaccharidiques tels que les triols, par exemple le glycérol, les tétraols, par exemple l'érythritol, les pentols, par exemple le xylitol, l'arabitol et le ribitol, les hexols, par exemple le sorbitol, le dulcitol et le mannitol, les heptols, par exemple le volemitol, ainsi que les polyols disaccharidiques tels que le maltitol, l'isomaltitol et le lactitol.

Selon un mode de réalisation de l'invention, le polyol ajouté dans la composition aqueuse du procédé de l'invention est du sorbitol.

On ajoute le polyol dans la composition aqueuse à raison d'au moins 1%, de préférence d'au moins 5% et de préférence encore d'au moins 10%, avec au plus 50%.

La composition peut également comprendre une autre macromolécule, appelée de façon générique « protéine de charge » même si des macromolécules autres que des protéines sont appropriées, laquelle n'a rien à voir avec la GDH mais est présente en large excès par rapport à la GDH, améliorant encore la stabilisation de la GDH. Cette «protéine de charge » a un effet bouclier en ce sens qu'elle va en partie subir les modifications physico-chimiques dans la solution aqueuse, permettant ainsi de protéger la molécule d'intérêt, ici la GDH. Cette macromolécule ajoutée peut par exemple être une protéine comme la BSA (Bovine Serum Albumine), ou encore des polymères synthétiques type dextran ou polyéthylène glycol. La BSA, par exemple, est ajoutée à raison de 50 g/L vs 3 mg/L de GDH.

La composition aqueuse est tamponnée pour avoir un pH compris entre 4,5 et 7, de préférence entre 5,5 et 6,5, un pH de 5,8 étant préféré. Comme indiqué précédemment, le composé de stabilisation de l'invention peut également être utilisé à titre de tampon, ou bien un autre composé tampon peut être ajouté.

Selon un mode de réalisation, les compositions aqueuses de l'invention comprennent la glutamate déshydrogénase d'une bactérie du genre *Clostridium* et un composé de stabilisation qui est un acide carboxylique ayant une chaîne carbonée d'au moins 3 atomes de carbone et comportant au moins 2 groupements -COOH ou un de ses sels, étant entendu que le composé de stabilisation n'est ni le glutamate, ni l'alpha-cétoglutarate, produit d'hydrolyse du glutamate par l'enzyme GDH et la coenzyme NAD+, ni le citrate, ni le succinate, ni le glutarate.

Selon un autre mode de réalisation, les compositions aqueuses de l'invention excluent aussi l'acide glutamique, l'acide alpha-cétoglutarique, l'acide glutarique, l'acide succinique et/ou l'acide citrique.

Selon un autre mode de réalisation, lorsque la bactérie du genre *Clostridium* est choisie parmi les espèces de *Clostridium difficile, Clostridium botulinum* et *Clostridium perfringens,* alors les compositions aqueuses comprenant la glutamate déshydrogénase d'une de ces bactéries et un composé de stabilisation qui est un acide carboxylique ayant une chaîne carbonée d'au moins 3 atomes de carbone et comportant au moins 2 groupements -COOH ou un de ses sels, étant entendu que le composé de stabilisation n'est ni le glutamate, ni l'alpha-cétoglutarate, ni le citrate.

Selon un autre mode de réalisation, les compositions aqueuses de l'invention excluent aussi l'acide glutamique, l'acide alpha-cétoglutarique et/ou l'acide citrique.

Les mêmes caractéristiques et préférences décrites précédemment, notamment quant au choix de la GDH, du composé de stabilisation, des composés à ajouter et de leur quantité relative, données en relation avec le procédé, s'appliquent également aux compositions aqueuses selon l'invention.

Les compositions aqueuses de l'invention, notamment celles obtenues selon le procédé de l'invention sont particulièrement utiles pour la détection de la présence d'une bactérie du genre *Clostridium* dans un échantillon biologique susceptible de contenir une telle bactérie.

Les échantillons biologiques susceptibles de contenir une bactérie du genre *Clostridium* peuvent être des échantillons issus du domaine clinique ou du domaine du contrôle d'innocuité, voire de stérilité des produits industriels. Dans le domaine clinique, l'échantillon est un prélèvement biologique animal, de préférence humain, tel que les selles ou dérivés, par exemple extrait de protéines fécales, urine, sang ou dérivés, par exemple sérum ou plasma, pus, etc. Dans le domaine industriel, l'échantillon provient d'un produit alimentaire ou cosmétique.

Le dosage immunologique qualitatif ou quantitatif de la GDH sera de préférence un dosage sandwich qui est un dosage largement connu de l'homme du métier mettant en œuvre deux partenaires de liaison à la GDH. L'un des deux partenaires peut être couplé à un marqueur pour former un conjugué ou un traceur. L'autre partenaire de liaison peut être capturé sur un support solide. On parle alors de partenaire de capture pour ce dernier et partenaire de détection pour le premier.

Le signal mesuré émis par le conjugué est alors proportionnel à la quantité de GDH de l'échantillon biologique.

Par marqueur, on entend, notamment, toute molécule contenant un groupement réactif avec un groupement du partenaire de liaison, directement sans modification chimique, ou après modification chimique pour inclure un tel groupement, laquelle molécule est capable de générer directement ou indirectement un signal détectable. Une liste non limitative de ces marqueurs de détection directe consiste en :
- les enzymes qui produisent un signal détectable par exemple par colorimétrie, fluorescence, luminescence, comme la peroxydase de raifort, la phosphatase alcaline, la β-galactosidase, la glucose-6-phosphate déshydrogénase,
- les chromophores comme les composés fluorescents, luminescents, colorants,
- les molécules radioactives comme le ³²P, le ³⁵S ou le ¹²⁵I,
- les molécules fluorescentes telles que les Alexa ou les phycocyanines, et
- les sels électrochimiluminescents tels que des dérivés organo-métalliques à base d'acridinium ou de ruthénium.

Des systèmes indirects de détection peuvent aussi être utilisés, comme par exemple des ligands capables de réagir avec un anti-ligand. Le ligand correspond alors au marqueur pour constituer, avec le partenaire de liaison, le conjugué.

Les couples ligand/anti-ligand sont bien connus de l'homme du métier, ce qui est le cas par exemple des couples suivants : biotine/streptavidine, haptène/anticorps, antigène/anticorps, peptide/anticorps, sucre/lectine, polynucléotide/complémentaire du polynucléotide.

L'anti-ligand peut alors être détectable directement par les marqueurs de détection directe décrits précédemment ou être lui-même détectable par un autre couple ligand/anti-ligand, et ainsi de suite.

Ces systèmes indirects de détection peuvent conduire, dans certaines conditions, à une amplification du signal. Cette technique d'amplification du signal est bien connue de l'homme du métier, et l'on pourra se reporter aux demandes de brevet antérieures FR 2781802 ou WO 95/08000 de la Demanderesse.

Selon le type de marquage utilisé, l'homme du métier ajoutera des réactifs permettant la visualisation du marquage ou l'émission d'un signal détectable par tout type d'appareil de mesure approprié, comme par exemple un spectrophotomètre, un spectrofluorimètre, un densitomètre ou encore une caméra haute définition.

Dans les procédés de dosage de la GDH, les compositions aqueuses de l'invention, le cas échéant obtenues selon le procédé de l'invention, sont particulièrement utiles pour l'établissement d'une gamme étalon, ce qui constitue un autre objet de l'invention. L'établissement de la gamme étalon, étape nécessaire pour pouvoir effectuer une quantification de la GDH, est une étape largement connue de l'homme du métier. En quelques mots, il consiste à mesurer le signal généré par des quantités ou concentrations croissantes et connues de l'analyte GDH, à tracer la courbe donnant le signal en fonction de la quantité ou de la concentration et à trouver un modèle mathématique qui représente de la manière la plus fidèle possible cette relation. Pour ce faire, plusieurs compositions aqueuses de l'invention sont utilisées, chacune contenant une concentration différente en GDH. Le modèle mathématique sera utilisé pour déterminer par extrapolation les quantités ou concentrations de GDH inconnues contenues dans l'échantillon biologique à tester.

Les compositions aqueuses de l'invention, le cas échéant obtenues selon le procédé de l'invention, sont également particulièrement utiles en tant que calibrateur, ce qui constitue un autre objet de l'invention. Dans ce cas, la concentration en GDH de la composition est fixe et connue. Le signal généré lors de l'utilisation de la trousse d'immunodosage par le calibrateur est également connu. Le calibrateur sert à vérifier que la mesure (signal) produite lors de la mise en œuvre de la trousse d'immunodosage correspond bien à la valeur attendue. Si ce n'est pas le cas, le calibrateur sert à mesurer la dérive qui pourra le cas échéant être corrigée mathématiquement ou par une intervention physique sur l'instrument de mesure (ajustement).

Les compositions aqueuses de l'invention, le cas échéant obtenues selon le procédé de l'invention, sont également particulièrement utiles en tant que contrôle, ce qui constitue un autre objet de l'invention. A ce titre, elles sont utilisées par exemple pour vérifier que la trousse d'immunodosage fonctionne selon les attentes (également appelé témoin ou contrôle positif) et que la détection de la GDH dans l'échantillon biologique n'est pas faussement négative dans la mesure où la méthode de détection a bien fonctionné avec la composition aqueuse de l'invention.

La détection de la GDH dans un échantillon biologique permet de conclure à la présence de la bactérie.

L'invention concerne un procédé de détection de la présence d'une bactérie du genre *Clostridium* dans un échantillon biologique susceptible de contenir une telle bactérie, caractérisé en ce qu'il comprend les étapes de (i) mettre un œuvre un procédé de détection de la présence d'une bactérie du genre *Clostridium* par détection ou quantification de glutamate déshydrogénase dans ledit échantillon en utilisant une composition aqueuse de l'invention, le cas échéant telle qu'obtenue selon le procédé de l'invention, et (ii) si le procédé de l'étape (i) est positif, conclure à la présence de la bactérie.

En d'autres termes, pour l'étape (ii) un résultat positif à l'étape (i) permet de conclure à la présence de la bactérie.

En revanche, cela ne renseigne en rien sur le fait que cette bactérie produit ou non au moins une toxine, ce qui est particulièrement important en tant qu'aide au diagnostic lorsqu'un patient présente des symptômes qui pourraient être provoqués par la présence d'une bactérie toxinogène et exprimant au moins une toxine.

Aussi, un autre objet de l'invention concerne un procédé de détection de la présence d'une bactérie toxinogène du genre *Clostridium* produisant au moins une toxine, dans un échantillon biologique susceptible de contenir une telle bactérie et une telle au moins une toxine, caractérisé en ce qu'il comprend les étapes de :
(i) mettre un œuvre un procédé de détection de la présence d'une bactérie du genre *Clostridium* par détection ou quantification de glutamate déshydrogénase dans ledit échantillon en utilisant une composition aqueuse de l'invention, le cas échéant telle qu'obtenue selon le procédé de l'invention, et
(ii) si le procédé de l'étape (i) est négatif, conclure à l'absence de la bactérie ou
(ii') si le procédé de l'étape (i) est positif, mettre en œuvre un procédé de détection ou quantification d'au moins une toxine susceptible d'être libérée par ladite bactérie du genre *Clostridium,* dans le même échantillon biologique, ou dans un nouvel échantillon biologique issu du même individu, et conclure au fait que la bactérie est toxinogène et produit ladite au moins une toxine si ladite au moins une toxine est présente.

En d'autres termes, pour l'étape (ii) un résultat négatif à l'étape (i) permet de conclure à l'absence de la bactérie et un résultat positif à l'étape (i) permet de conclure à la présence de la bactérie.

L'étape (i) ci-dessus consistant à détecter la présence d'une bactérie du genre *Clostridium* par détection ou quantification de glutamate déshydrogénase dans l'échantillon biologique a été décrite précédemment. La composition aqueuse de l'invention, le cas échéant obtenue par le procédé de l'invention, peut alors être utilisée pour l'établissement de la gamme étalon et/ou à titre de calibrateur et/ou à titre de contrôle positif.

L'étape (ii') ci-dessus consistant à détecter ou quantifier au moins une toxine susceptible d'être libérée par ladite bactérie du genre *Clostridium* est une étape largement connue de l'homme du métier. Une telle détection ou quantification peut être mise en œuvre par exemple par immunodosage en utilisant des partenaires de liaison aux toxines recherchées. L'immunodosage des toxines est mis en œuvre de façon similaire à l'immunodosage de la GDH tel que décrit précédemment. Des kits d'immunodosage de toxine de bactéries du genre *Clostridium* sont disponibles sur le marché, comme par exemple le kit VIDAS® *Clostridium difficile* A&B qui permet de détecter les toxines A et B de *Clostridium difficile.*

La spectrométrie de masse pourra également être utilisée pour réaliser l'étape de détection/quantification de la toxine. Cette technique est une technique d'analyse qui permet la détermination de la masse molaire des composés analysés, ainsi que l'identification de leur structure moléculaire, voire leur quantification. Appliquée à un mélange complexe comme un fluide biologique ou des selles, elle nécessite d'être couplée à une technique séparative qui permet d'en réduire la complexité. Il s'agit, le plus souvent, de la chromatographie en phase gazeuse (GC) ou de la chromatographie en phase liquide (LC). La spectrométrie de masse en tandem (MS/MS) combine 2 analyseurs et pourra être utilisée aux fins de détection/quantification. Les composés ioniques sélectionnés dans le premier analyseur puis fragmentés sont analysés de manière plus fine dans le second. Cette double analyse permet d'augmenter de manière significative la spécificité de la méthode. Pour cette technologie, on pourra notamment se référer à Van den Broek *et al.,* 2013.

La détection et/ou quantification des toxines peut également être mise en œuvre par un test d'immunotoxicité dans les selles (CTA) qui permet de mettre en évidence les effets biologiques des toxines dans les selles (Eckert C. *et al.,* 2011).

La détection ou quantification de la toxine est effectuée dans le même échantillon biologique que celui utilisé pour la détection ou quantification de la GDH, dont une partie a été gardée à cet égard, ou bien dans un nouvel échantillon biologique issu de la même provenance, c'est-à-dire de l'individu dont est issu le premier échantillon biologique testé par rapport à la présence de GDH si l'échantillon biologique est un échantillon clinique ou de la même source si l'échantillon biologique est un échantillon industriel. Le deuxième échantillon biologique est soit de même nature, ou de nature différente, le premier cas étant préféré.

Selon un mode de réalisation particulier, la bactérie toxinogène dont on veut détecter la présence est *Clostridium difficile* et ladite au moins une toxine comprend la toxine A, la toxine B ou les deux.

D'autres bactéries toxinogènes du genre *Clostridium* ont été décrites précédemment.

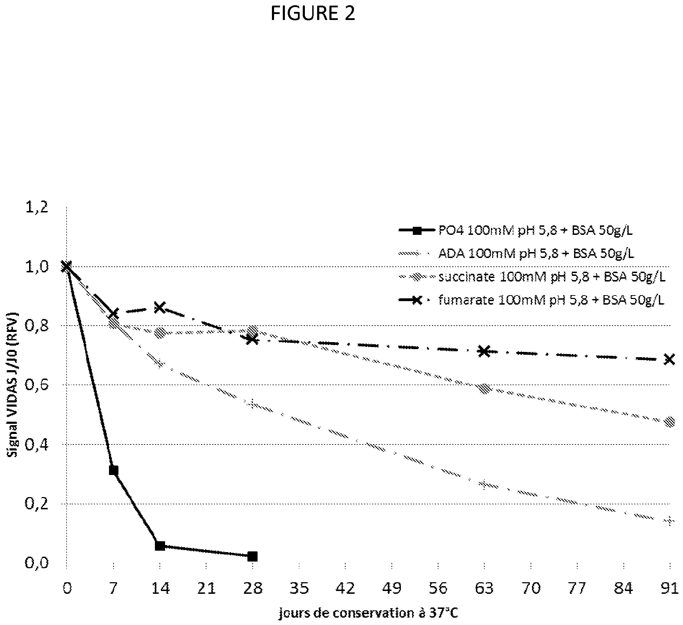

L'invention sera mieux comprise à l'aide des exemples suivants qui sont donnés à titre illustratif et non limitatif, ainsi qu'à l'aide des figures 1 et 2, dans lesquelles :
- la Figure 1 donne des exemples de composés de stabilisation mis en œuvre dans les compositions aqueuses selon l'invention, ainsi que leur formule chimique, et
- la Figure 2 est un graphe donnant l'évolution du signal fluorescent obtenu lors d'un immunodosage avec la trousse VIDAS® GDH (bioMérieux) émises par des compositions aqueuses selon l'invention ou des compositions comparatives, en fonction du temps, lorsque ces compositions sont maintenues à 37°C.

### EXEMPLES

### Exemple 1 : Préparation de GDH recombinante de Clostridium difficile

Le gène *gluD* codant pour la GDH de *Clostridium difficile* (N° accession Genbank M65250) complété d'une séquence codant un tag HIS est cloné dans le vecteur pMR78 (bioMérieux, France). Le plasmide d'expression ainsi construit est introduit dans des bactéries *E. coli* BL21 et dérivées (Stratagene, Agilent Technologies). Les cultures sont réalisées en milieu 2x YT (Difco), en présence d'ampicilline, à 37°C sous agitation. L'induction de l'expression de la protéine se fait par addition de 1 mM d'IPTG (isopropyl beta-D-1-thiogalactopyranoside). Les bactéries sont collectées par centrifugation en fin de culture.

Les culots bactériens sont repris en tampon PBS 2X (phosphate buffered saline) et lysés. Les lysats sont centrifugés à 3000 g pendant 30 min à 4°C. Le surnageant contient les protéines solubles dont la GDH recombinante à purifier.

La purification de la protéine se fait par chromatographie d'affinité métal chélate en une étape. Le surnageant obtenu après centrifugation est chargé sur une résine Ni-NTA-Agarose (Qiagen). Après un cycle de lavage, la protéine est éluée en présence d'un gradient imidazole. La protéine est dialysée dans un tampon phosphate 20 mM.

### Exemple 2 : Mise en évidence de la stabilisation des propriétés antigéniques de la GDH en utilisant des composés de stabilisation selon l'invention

La GDH recombinante, préparée dans l'exemple 1, est diluée à 3ng/mL dans les formulations suivantes, selon les indications relatives au calibrateur et contrôle (S1/C1) données dans la notice du réactif VIDAS® GDH (Ref 30125, bioMérieux, France) :
- Composition comparative : phosphate 100mM + BSA 50g/L, pH 5,8 (formulation de la solution de calibration du kit Vidas®, GDH sous forme lyophilisée, reprise dans de l'eau déminéralisée),
- Composition selon l'invention 1 : acide N-(2-acétamido)iminodiacétique (ADA) 100mM + BSA 50g/L pH 5.8 (composition ADA)
- Composition selon l'invention 2 : acide succinique 100mM + BSA 50g/L pH 5.8 (composition succinate)
- Composition selon l'invention 3 : fumarate disodique 100mM + BSA 50g/L pH 5.8 (composition fumarate).

Chaque composition (comparative et ADA, succinate et fumarate) a été préparée au préalable comme suit : chaque composé de stabilisation (1,18 g d'acide succinique - Merck, 1,6 g de fumarate de sodium dibasique - Sigma, 1,90 g d'ADA - Sigma ou 0,78 g de phosphate NaH₂PO₄.2H₂O + 1,79 g de Na₂HPO₄.12H₂O) a été mélangé à de l'eau déminéralisée pour obtenir 50 mL. Puis du NaOH 10M N a été ajouté pour ajuster le pH à 5,8. Cinq g de BSA (Millipore) ont été ajoutés. Enfin, de l'eau déminéralisée a été ajoutée pour obtenir une solution de 100 mL.

Les 4 compositions contenant la GDH sont ensuite aliquotées en fractions de 1 mL puis conservées à 37+/-1°C. L'impact de la formulation sur la stabilité des propriétés antigéniques de la protéine GDH recombinante est évaluée en réalisant plusieurs dosages de la GDH sur une période de 91 jours avec le kit VIDAS® GDH (Ref 30125) et l'instrument VIDAS® selon les instructions du fabricant (bioMérieux, France).

La mise en œuvre du test VIDAS GDH respecte le protocole du kit commercialisé :
- introduction de 200µL d'échantillon + 1mL du réactif de prétraitement R1
- homogénéisation par vortex
- introduction de 300µL de cette dilution au 1/6 dans le puits échantillon de la cartouche du kit VIDAS® GDH

Chaque aliquot n'est utilisé que pour un seul temps de suivi, mais utilisé en duplicate systématiquement. L'instrument VIDAS mesure un signal de fluorescence et les résultats sont exprimés en « relative fluorescence value » ou RFV.

Les résultats de RFV obtenus pour les 2 mesures en duplicate (1 et 2) ainsi que le ratio J/JO (RFV au jour J sur RFV au jour 0) sont donnés dans le tableau 4 ci-dessous et sont également reproduits sur le graphe de la Figure 2.

**Tableau 4**

| Jours 37°C | Composition comparative | | | Composition ADA | | | Composition succinate | | | Composition fumarate | | |
|---|---|---|---|---|---|---|---|---|---|---|---|---|
| | 1 | 2 | J/JO | 1 | 2 | J/JO | 1 | 2 | J/JO | 1 | 2 | J/JO |
| 0 | 417 | 445 | 1 | 428 | 451 | 1 | 419 | 462 | 1 | 446 | 428 | 1 |
| 7 | 132 | 137 | 0,31 | 355 | 357 | 0,81 | 339 | 371 | 0,81 | 371 | 364 | 0,84 |
| 14 | 25 | 25 | 0,06 | 278 | 311 | 0,67 | 343 | 340 | 0,78 | 375 | 378 | 0,86 |
| 28 | 8 | 12 | 0,02 | 249 | 221 | 0,53 | 336 | 354 | 0,78 | 329 | 329 | 0,75 |
| 63 | NA | NA | NA | 124 | 108 | 0,26 | 258 | 260 | 0,59 | 322 | 302 | 0,71 |
| 91 | NA | NA | NA | 59 | 64 | 0,14 | 209 | 210 | 0,48 | 297 | 302 | 0,69 |

| | | | | | | | | | | | | |
|---|---|---|---|---|---|---|---|---|---|---|---|---|
| NA = non applicable | | | | | | | | | | | | |

Les résultats mettent en évidence qu'à 37°C, l'utilisation de composés de stabilisation consistant en des acides carboxyliques ayant une chaîne carbonée d'au moins 3 atomes de carbone et comportant au moins 2 groupements -COOH, permet d'améliorer très sensiblement la durée de conservation d'une solution aqueuse contenant de la GDH puisque, pour la composition comparative, il n'y a plus de signal à 28 jours, tandis que le rapport J/JO à cette date pour les compositions selon l'invention est au moins égal à 0,5.

### EXEMPLE 3 : Suivi des propriétés antigéniques de la GDH en solution aqueuse en utilisant l'acide citrique comme composé de stabilisation

On a répété le mode opératoire de l'exemple 2, à ceci près qu'on a utilisé l'acide citrique (100 mM) comme composé de stabilisation et qu'on a conservé les solutions aqueuses sur une période plus longue, à 2-8°C et à 37°C.

Les résultats de RFV obtenus pour les 2 mesures en duplicate (1 et 2) ainsi que le ratio J/JO sont donnés dans le tableau 5 ci-dessous.

**Tableau 5**

| Jours | Composition Citrate - 37°C | | | Composition Citrate - 2-8°C | | |
|---|---|---|---|---|---|---|
| | 1 | 2 | J/JO | 1 | 2 | J/JO |
| 0 | 713 | 677 | 1 | 713 | 677 | 1 |
| 1 | 697 | 757 | 1,05 | 653 | 722 | 0,99 |
| 7 | 717 | 683 | 1 | 636 | 694 | 0,96 |
| 14 | 666 | 678 | 0,97 | 646 | 700 | 0,97 |
| 29 | 521 | 565 | 0,78 | 692 | 680 | 0,99 |
| 51 | 421 | 418 | 0,6 | 613 | 641 | 0,9 |
| 78 | 233 | 222 | 0,33 | 701 | 680 | 0,99 |
| 124 | 67 | 67 | 0,1 | 648 | 690 | 0,96 |
| 184 | 6 | 10 | 0,01 | 739 | 774 | 1,09 |
| 275 | NA | NA | NA | 657 | 663 | 0,95 |
| 369 | NA | NA | NA | 664 | 612 | 0,92 |
| 552 | NA | NA | NA | 523 | 558 | 0,78 |

| | | | | | | |
|---|---|---|---|---|---|---|
| NA : non applicable | | | | | | |

Les résultats ci-dessus mettent en évidence que l'ajout d'acide citrique permet une très bonne stabilité liée à la conservation des propriétés antigéniques de la GDH, avec une stabilisation quasi optimale, même au bout de 18 mois, lorsque la composition aqueuse est conservée entre 2-8°C.

### EXEMPLE 4 : Suivi des propriétés antigéniques de la GDH en solution aqueuse en utilisant de l'acide succinique et du sorbitol

On a répété le mode opératoire de l'exemple 2, à ceci près qu'on a ajouté en plus 10% de sorbitol dans une composition succinate et qu'on a conservé les solutions aqueuses sur une période plus longue, à 2-8°C et à 37°C.

Les résultats de RFV obtenus pour les 2 mesures en duplicate (1 et 2) ainsi que le ratio J/JO sont donnés dans le tableau 6 ci-dessous.

**Tableau 6**

| | | | | | | |
|---|---|---|---|---|---|---|
| Jours | Composition Succinate avec sorbitol - 37°C | | | Composition Succinate avec Sorbitol - 2-8°C | | |

| | 1 | 2 | J/JO | 1 | 2 | J/JO |
|---|---|---|---|---|---|---|
| 0 | 726 | 710 | 1 | 726 | 710 | 1 |
| 1 | 740 | 714 | 1 | 744 | 700 | 1,01 |
| 7 | 702 | 696 | 0,97 | 725 | 764 | 1,04 |
| 14 | 689 | 655 | 0,94 | 720 | 765 | 1,03 |
| 29 | 624 | 658 | 0,89 | 690 | 708 | 0,97 |
| 51 | 653 | 646 | 0,9 | 603 | 668 | 0,89 |
| 78 | 606 | 591 | 0,83 | 670 | 720 | 0,97 |
| 124 | 529 | 556 | 0,76 | 709 | 691 | 0,97 |
| 184 | 379 | 376 | 0,53 | 656 | 785 | 1 |
| 275 | 267 | 286 | 0,39 | 659 | 710 | 0,95 |
| 369 | 156 | 161 | 0,22 | 692 | 663 | 0,94 |
| 552 | 48 | 48 | 0,07 | 663 | 710 | 0,96 |
| 891 | NC | NC | NC | 638 | 681 | 0,92 |

| | | | | | | |
|---|---|---|---|---|---|---|
| NC : non calculé | | | | | | |

Les résultats ci-dessus mettent en évidence que l'acide succinique permet également une stabilisation longue des propriétés antigéniques de la GDH en solution aqueuse, l'ajout de sorbitol n'altérant pas cette stabilisation, avec une stabilisation quasi optimale, même au bout d'environ 30 mois, lorsque la solution aqueuse est conservée entre 2-8°C.

### Références Bibliographiques

- Anderson BM et al, 1993, Archives of Biochemistry and Biophysics, 300(1) : 483-488
- Boersma YL, Plückthun A, 2011, Curr. Opin. Biotechnol, 22 : 849-857
- Eckert C. et al., 2011, Journal des Anti-Infectieux, 13(2) : 109-116
- Ellington AD et Szostak JW., 1990, Nature, 346 : 818-822
- Garcia-Galan C. et al., 2013, Enzyme and Microbial Technology, 52(4-5) : 211-217
- Van den Broek et al., 2013, J. Chromatogr. B, 929 : 161-179

## Revendications

1. Procédé de détection de la présence d'une bactérie du genre *Clostridium* dans un échantillon biologique susceptible de contenir une telle bactérie, **caractérisé en ce qu'**il comprend les étapes de :
(i) mettre un œuvre un procédé de détection de la présence d'une bactérie du genre *Clostridium* par détection ou quantification de la présence de glutamate déshydrogénase dans ledit échantillon en utilisant une composition aqueuse comprenant la glutamate déshydrogénase d'une bactérie du genre *Clostridium* et un composé de stabilisation qui est un acide carboxylique ayant une chaîne carbonée d'au moins 3 atomes de carbone et comportant au moins 2 groupements -COOH ou un de ses sels, et
(ii) un résultat positif à l'étape (i) permet de conclure à la présence de la bactérie.

2. Procédé selon la revendication 1, **caractérisé en ce que** la composition comprend également une macromolécule, également appelée « protéine de charge », et/ou du polyol.

3. Procédé selon l'une des revendications 1 ou 2, **caractérisé en ce que** l'acide carboxylique contient une chaîne carbonée de 4, 5 ou 6 atomes de carbone.

4. Procédé selon l'une quelconque des revendications précédentes, **caractérisé en ce que** le composé de stabilisation est choisi parmi les acides carboxyliques suivants et leurs sels :
(i) les acides carboxyliques contenant une chaîne carbonée de 4 atomes de carbone ayant au moins 2 groupements -COOH et des groupements -CX- choisis indépendamment parmi =CH-, -CH₂- et -C(H)OH-, et
(ii) les acides carboxyliques contenant une chaîne carbonée de 5 atomes de carbone ayant au moins 2 groupements -COOH et des groupements -CX- choisis parmi -CH₂- et -C(H)NH₂, et
(iii) les acides carboxyliques contenant une chaîne carbonée de 6 atomes de carbone ayant au moins 2 groupements -COOH et des groupements « CX » choisis parmi -CH₂-, -C(H)OH- et -C(O)NH₂.

5. Procédé selon l'une quelconque des revendications précédentes, **caractérisé en ce que** l'acide carboxylique contient dans sa chaîne carbonée une double liaison et est un isomère E.

6. Procédé selon l'une quelconque des revendications précédentes, **caractérisé en ce que** l'acide carboxylique contient 2 groupements -COOH.

7. Procédé selon l'une quelconque des revendications précédentes, **caractérisé en ce que** le composé de stabilisation est choisi parmi l'acide fumarique, l'acide succinique, l'acide malique, l'acide glutarique, l'acide citrique, l'acide tartrique, l'acide N-(2-acétamido)iminodiacétique, l'acide glutamique, l'acide adipique, l'acide aspartique, l'acide pimélique et l'acide malonique et leurs sels.

8. Procédé selon la revendication 7, **caractérisé en ce que** le composé de stabilisation est choisi parmi l'acide succinique et l'acide fumarique et leurs sels, en particulier de métal alcalin.

9. Procédé selon l'une quelconque des revendications précédentes, **caractérisée en ce que** la glutamate déshydrogénase est une enzyme de la bactérie de l'espèce *Clostridium difficile.*

10. Procédé de détection de la présence d'une bactérie toxinogène du genre *Clostridium* produisant au moins une toxine, dans un échantillon biologique susceptible de contenir une telle bactérie et une telle au moins une toxine, **caractérisé en ce qu'**il comprend les étapes de :
(i) mettre un œuvre un procédé de détection de la présence d'une bactérie du genre *Clostridium* par détection ou quantification de la présence de glutamate déshydrogénase dans ledit échantillon en utilisant une composition aqueuse comprenant la glutamate déshydrogénase d'une bactérie du genre *Clostridium* et un composé de stabilisation qui est un acide carboxylique ayant une chaîne carbonée d'au moins 3 atomes de carbone et comportant au moins 2 groupements -COOH ou un de ses sels, et
(ii) un résultat négatif à l'étape (i) permet de conclure à l'absence de la bactérie ou
(ii') si le procédé de l'étape (i) est positif, mettre en œuvre un procédé de détection ou quantification d'au moins une toxine susceptible d'être libérée par ladite bactérie du genre *Clostridium,* dans le même échantillon biologique, ou dans un nouvel échantillon biologique issu du même individu, et conclure au fait que la bactérie est toxinogène et produit ladite au moins une toxine si ladite au moins une toxine est présente.

11. Procédé selon la revendication 10, **caractérisé en ce que** la bactérie est *Clostridium difficile* et ladite au moins une toxine comprend la toxine A, la toxine B ou les deux.

## Patentansprüche

1. Verfahren zum Nachweis des Vorliegens eines Bakteriums der Gattung *Clostridium* in einer biologischen Probe, welche ein solches Bakterium enthalten könnte, **dadurch gekennzeichnet, dass** es die folgenden Schritte umfasst:
(i) Durchführen eines Verfahrens zum Nachweis des Vorliegens eines Bakteriums der Gattung *Clostridium,* indem das Vorliegen von Glutamat-Dehydrogenase in der Probe nachgewiesen oder quantifiziert wird, wobei dazu eine wässrige Zusammensetzung verwendet wird, welche die Glutamat-Dehydrogenase eines Bakteriums der Gattung *Clostridium* sowie eine stabilisierende Verbindung umfasst, bei welcher es sich um eine Carbonsäure mit einer Kohlenstoffkette aus mindestens 3 Kohlenstoffatomen, die weiterhin mindestens 2 COOH-Gruppen aufweist, oder um eines von deren Salzen handelt,
und
(ii) ein positives Ergebnis in Schritt (i) ermöglicht die Schlussfolgerung, dass das Bakterium vorliegt.

2. Verfahren nach Anspruch 1, **dadurch gekennzeichnet, dass** die Zusammensetzung weiterhin ein Makromolekül, welches auch als "Ladungsprotein" bezeichnet wird, und/oder Polyol umfasst.

3. Verfahren nach einem der Ansprüche 1 oder 2, **dadurch gekennzeichnet, dass** die Carbonsäure eine Kohlenstoffkette aus 4, 5 oder 6 Kohlenstoffatomen enthält.

4. Verfahren nach einem beliebigen der vorhergehenden Ansprüche, **dadurch gekennzeichnet, dass** die stabilisierende Verbindung aus den folgenden Carbonsäuren und deren Salzen ausgewählt ist:
(i) den Carbonsäuren, die eine Kohlenstoffkette aus 4 Kohlenstoffatomen enthalten, welche mindestens 2 COOH-Gruppen sowie -CX-Gruppen aufweist, wobei diese unabhängig voneinander aus =CH-, -CH₂- und -C(H)OH-ausgewählt sind, und
(ii) den Carbonsäuren, die eine Kohlenstoffkette aus 5 Kohlenstoffatomen enthalten, welche mindestens 2 COOH-Gruppen sowie -CX-Gruppen aufweist, wobei diese unabhängig voneinander aus -CH₂- und -C(H)NH₂ ausgewählt sind, und
(iii) den Carbonsäuren, die eine Kohlenstoffkette aus 6 Kohlenstoffatomen enthalten, welche mindestens 2 COOH-Gruppen sowie "CX"-Gruppen aufweist, wobei diese unabhängig voneinander aus -CH₂-, -C(H)OH- und -C(O)NH₂ ausgewählt sind.

5. Verfahren nach einem beliebigen der vorhergehenden Ansprüche, **dadurch gekennzeichnet, dass** die Carbonsäure in ihrer Kohlenstoffkette eine Doppelbindung enthält und es sich um ein E-Isomer handelt.

6. Verfahren nach einem beliebigen der vorhergehenden Ansprüche, **dadurch gekennzeichnet, dass** die Carbonsäure 2 COOH-Gruppen enthält.

7. Verfahren nach einem beliebigen der vorhergehenden Ansprüche, **dadurch gekennzeichnet, dass** die stabilisierende Verbindung aus Fumarsäure, Bernsteinsäure, Äpfelsäure, Glutarsäure, Citronensäure, Weinsäure, N-(2-Acetamido)iminodiessigsäure, Glutaminsäure, Adipinsäure, Asparaginsäure, Pimelinsäure und Malonsäure sowie deren Salzen ausgewählt ist.

8. Verfahren nach Anspruch 7, **dadurch gekennzeichnet, dass** die stabilisierende Verbindung aus Bernsteinsäure und Fumarsäure sowie deren Salzen, insbesondere mit Alkalimetallen, ausgewählt ist.

9. Verfahren nach einem beliebigen der vorhergehenden Ansprüche, **dadurch gekennzeichnet, dass** es sich bei der Glutamat-Dehydrogenase um ein Enzym des Bakteriums der Art *Clostridium difficile* handelt.

10. Verfahren zum Nachweis des Vorliegens eines toxinbildenden Bakteriums der Gattung *Clostridium,* das mindestens ein Toxin produziert, in einer biologischen Probe, welche ein derartiges Bakterium und mindestens ein derartiges Toxin enthalten könnte, **dadurch gekennzeichnet, dass** es die folgenden Schritte umfasst:
(i) Durchführen eines Verfahrens zum Nachweis des Vorliegens eines Bakteriums der Gattung *Clostridium,* indem das Vorliegen von Glutamat-Dehydrogenase in der Probe nachgewiesen oder quantifiziert wird, wobei dazu eine wässrige Zusammensetzung verwendet wird, welche die Glutamat-Dehydrogenase eines Bakteriums der Gattung *Clostridium* sowie eine stabilisierende Verbindung umfasst, bei welcher es sich um eine Carbonsäure mit einer Kohlenstoffkette aus mindestens 3 Kohlenstoffatomen, die weiterhin mindestens 2 COOH-Gruppen aufweist, oder um eines von deren Salzen handelt,
und
(ii) ein negatives Ergebnis in Schritt (i) ermöglicht die Schlussfolgerung, dass das Bakterium nicht vorliegt, oder
(ii') wenn das Verfahren des Schrittes (i) positiv ist, Durchführen eines Verfahrens zum Nachweis oder zur Quantifizierung mindestens eines Toxins, welches von dem Bakterium der Gattung *Clostridium* freigesetzt werden kann, in derselben biologischen Probe oder in einer neuen biologischen Probe, die von demselben Individuum stammt, und Schlussfolgern, dass das Bakterium toxinbildend ist und das mindestens eine Toxin produziert, wenn das mindestens eine Toxin vorliegt.

11. Verfahren nach Anspruch 10, **dadurch gekennzeichnet, dass** es sich bei dem Bakterium um *Clostridium difficile* handelt und das mindestens eine Toxin das Toxin A, das Toxin B oder diese beiden umfasst.

## Claims

1. A process for detecting the presence of a bacterium of the *Clostridium* genus in a biological sample that may contain such a bacterium, **characterized in that** it comprises the steps of:
(i) carrying out a process for detecting the presence of a bacterium of the *Clostridium* genus by detecting or quantifying the presence of glutamate dehydrogenase in said sample using an aqueous composition comprising glutamate dehydrogenase from a bacterium of the *Clostridium* genus and a stabilizing compound which is a carboxylic acid having a carbon-based chain of at least 3 carbon atoms and comprising at least 2 -COOH groups, or a salt thereof, and (ii) a positive result in step (i) makes it possible to conclude that the bacterium is present.

2. The process as claimed in claim 1, **characterized in that** the composition also comprises a macro molecule, referred to as a "filler protein", and/or polyol.

3. The process as claimed in claim 1 or 2, **characterized in that** the carboxylic acid contains a carbon-based chain of 4, 5 or 6 carbon atoms.

4. The process as claimed in any one of the preceding claims, **characterized in that** the stabilizing compound is chosen from the following carboxylic acids and salts thereof:
(i) carboxylic acids containing a carbon-based chain of 4 carbon atoms having at least 2 -COOH groups and -CX- groups independently chosen from =CH-, -CH₂- and - C(H)OH-, and
(ii) carboxylic acids containing a carbon-based chain of 5 carbon atoms having at least 2 -COOH groups and -CX- groups chosen from -CH₂- and -C(H)NH₂, and
(iii) carboxylic acids containing a carbon-based chain of 6 carbon atoms having at least 2 -COOH groups and "CX" groups chosen from -CH₂-, -C(H)OH- and -C(O)NH₂.

5. The process as claimed in any one of the preceding claims, **characterized in that** the carboxylic acid contains a double bond in its carbon-based chain and is an E isomer.

6. The process as claimed in any one of the preceding claims, **characterized in that** the carboxylic acid contains two -COOH groups.

7. The process as claimed in any one of the preceding claims, **characterized in that** the stabilizing compound is chosen from fumaric acid, succinic acid, malic acid, glutaric acid, citric acid, tartaric acid, N-(2-acetamido)iminodiacetic acid, glutamic acid, adipic acid, aspartic acid, pimelic acid and malonic acid, and salts thereof.

8. The process as claimed in claim 7, **characterized in that** the stabilizing compound is chosen from succinic acid and fumaric acid, and salts thereof, in particular alkali metal salts.

9. The process as claimed in any one of the preceding claims, **characterized in that** the glutamate dehydrogenase is an enzyme from the bacterium of the species *Clostridium difficile.*

10. A process for detecting the presence of a toxigenic bacterium of the *Clostridium* genus which produces at least one toxin, in a biological sample that may contain such a bacterium and at least one such toxin, **characterized in that** it comprises the steps of:
(i) carrying out a process for detecting the presence of a bacterium of the *Clostridium* genus by detecting or quantifying the presence of glutamate dehydrogenase in said sample using an aqueous composition comprising glutamate dehydrogenase from a bacterium of the *Clostridium* genus and a stabilizing compound which is a carboxylic acid having a carbon-based chain of at least 3 carbon atoms and comprising at least 2 -COOH groups, or a salt thereof, and
(ii) a negative result in step (i) makes it possible to conclude that the bacterium is absent, or
(ii') if the process of step (i) is positive, carrying out a process for detecting or quantifying at least one toxin that may be released by said bacterium of the *Clostridium* genus, in the same biological sample, or in a new biological sample from the same individual, and concluding that the bacterium is toxigenic and produces said at least one toxin if said at least one toxin is present.

11. The process as claimed in claim 10, **characterized in that** the bacterium is *Clostridium difficile* and said at least one toxin comprises toxin A, toxin B or both.


### SEQUENCE LISTING

<110> bioMérieux
<120> Satbilisation de la GDH en solution aqueuse
<130> STABGLU
<150> FR13 62354
   <151> 2013-12-10
<160> 3
<170> PatentIn version 3.5
<210> 1
   <211> 421
   <212> PRT
   <213> Clostridium difficile
<400> 1
   Met Ser Gly Lys Asp Val Asn Val Phe Glu Met Ala Gln Ser Gln Val
   1               5                   10                  15
   Lys Asn Ala Cys Asp Lys Leu Gly Met Glu Pro Ala Val Tyr Glu Leu
               20                  25                  30
   Leu Lys Glu Pro Met Arg Val Ile Glu Val Ser Ile Pro Val Lys Met
           35                  40                  45
   Asp Asp Gly Ser Ile Lys Thr Phe Lys Gly Phe Arg Ser Gln His Asn
       50                  55                  60
   Asp Ala Val Gly Pro Thr Lys Gly Gly Ile Arg Phe His Gln Asn Val
   65                  70                  75                  80
   Ser Arg Asp Glu Val Lys Ala Leu Ser Ile Trp Met Thr Phe Lys Cys
                   85                  90                  95
   Ser Val Thr Gly Ile Pro Tyr Gly Gly Gly Lys Gly Gly Ile Ile Val
               100                 105                 110
   Asp Pro Ser Thr Leu Ser Gln Gly Glu Leu Glu Arg Leu Ser Arg Gly
           115                 120                 125
   Tyr Ile Asp Gly Ile Tyr Lys Leu Ile Gly Glu Lys Val Asp Val Pro
       130                 135                 140
   Ala Pro Asp Val Asn Thr Asn Gly Gln Ile Met Ser Trp Met Val Asp
   145                 150                 155                 160
   Glu Tyr Asn Lys Leu Thr Gly Gln Ser Ser Ile Gly Val Ile Thr Gly
                   165                 170                 175
   Lys Pro Val Glu Phe Gly Gly Ser Leu Gly Arg Thr Ala Ala Thr Gly
               180                 185                 190
   Phe Gly Val Ala Val Thr Ala Arg Glu Ala Ala Ala Lys Leu Gly Ile
           195                 200                 205
   Asp Met Lys Lys Ala Lys Ile Ala Val Gln Gly Ile Gly Asn Val Gly
       210                 215                 220
   Ser Tyr Thr Val Leu Asn Cys Glu Lys Leu Gly Gly Thr Val Val Ala
   225                 230                 235                 240
   Met Ala Glu Trp Cys Lys Ser Glu Gly Ser Tyr Ala Ile Tyr Asn Glu
                   245                 250                 255
   Asn Gly Leu Asp Gly Gln Ala Met Leu Asp Tyr Met Lys Glu His Gly
               260                 265                 270
   Asn Leu Leu Asn Phe Pro Gly Ala Lys Arg Ile Ser Leu Glu Glu Phe
           275                 280                 285
   Trp Ala Ser Asp Val Asp Ile Val Ile Pro Ala Ala Leu Glu Asn Ser
       290                 295                 300
   Ile Thr Lys Glu Val Ala Glu Ser Ile Lys Ala Lys Leu Val Cys Glu
   305                 310                 315                 320
   Ala Ala Asn Gly Pro Thr Thr Pro Glu Ala Asp Glu Val Phe Ala Glu
                   325                 330                 335
   Arg Gly Ile Val Leu Thr Pro Asp Ile Leu Thr Asn Ala Gly Gly Val
               340                 345                 350
   Thr Val Ser Tyr Phe Glu Trp Val Gln Asn Leu Tyr Gly Tyr Tyr Trp
           355                 360                 365
   Ser Glu Glu Glu Val Glu Gln Lys Glu Glu Ile Ala Met Val Lys Ala
       370                 375                 380
   Phe Glu Ser Ile Trp Lys Ile Lys Glu Glu Tyr Asn Val Thr Met Arg
   385                 390                 395                 400
   Glu Ala Ala Tyr Met His Ser Ile Lys Lys Val Ala Glu Ala Met Lys
                   405                 410                 415
   Leu Arg Gly Trp Tyr
   420
<210> 2
   <211> 448
   <212> PRT
   <213> Clostridium perfringens
<400> 2
   Met Glu Val Lys Lys Tyr Val Asp Asn Leu Met Glu Asp Leu Lys Lys
   1 5 10 15
   Asn Asn Pro Gly Glu Ser Glu Phe Leu Ala Ala Ala Glu Glu Val Leu
   20 25 30
   Tyr Ser Leu Val Pro Val Leu Glu Lys Asn Pro Lys Tyr Met Glu Glu
   35 40 45
   Gly Ile Leu Glu Arg Ile Val Glu Pro Glu Arg Val Ile Met Phe Arg
   50 55 60
   Val Pro Trp Val Asp Asp Ala Gly Asn Val Arg Val Asn Arg Gly Tyr
   65 70 75 80
   Arg Val Gln Phe Asn Ser Ala Ile Gly Pro Tyr Lys Gly Gly Leu Arg
   85 90 95
   Phe His Pro Ser Val Asn Leu Ser Ile Ile Lys Phe Leu Gly Phe Glu
   100 105 110
   Gln Ile Phe Lys Asn Ser Leu Thr Thr Leu Pro Ile Gly Gly Gly Lys
   115 120 125
   Gly Gly Ser Asn Phe Asp Pro Lys Gly Lys Ser Asp Arg Glu Ile Met
   130 135 140
   Arg Phe Cys Gln Ser Phe Met Ser Glu Leu Tyr Arg His Ile Gly Pro
   145 150 155 160
   Asn Thr Asp Val Pro Ala Gly Asp Ile Gly Val Gly Gly Arg Glu Ile
   165 170 175
   Gly Tyr Met Phe Gly Gln Tyr Lys Lys Leu Lys Asn Ser Val Asp Ala
   180 185 190
   Gly Val Leu Thr Gly Lys Gly Leu Thr Tyr Gly Gly Ser Leu Ala Arg
   195 200 205
   Lys Glu Ala Thr Gly Tyr Gly Leu Val Tyr Phe Val Asp Glu Met Leu
       210                 215                 220
   Arg Asp Asn Gly Gln Thr Ile Glu Gly Lys Thr Val Val Ile Ser Gly
   225                 230                 235                 240
   Ser Gly Asn Val Ala Ile Tyr Ala Thr Glu Lys Val Gln Glu Leu Gly
                   245                 250                 255
   Gly Lys Val Val Ala Leu Ser Asp Ser Ser Gly Tyr Val Tyr Asp Glu
               260                 265                 270
   Asn Gly Ile Asp Leu Glu Val Val Lys Glu Ile Lys Glu Val Lys Arg
           275                 280                 285
   Gly Arg Ile Ser Glu Tyr Val Asn Tyr Val Lys Thr Ala Lys Phe Thr
       290                 295                 300
   Glu Gly Phe Arg Gly Ile Trp Asn Val Lys Cys Asp Ile Ala Leu Pro
   305                 310                 315                 320
   Cys Ala Thr Gln Asn Glu Ile Asp Lys Ser Ser Ala Lys Thr Leu Ile
                   325                 330                 335
   Asp Asn Gly Val Ile Ala Val Gly Glu Gly Ala Asn Met Pro Ser Thr
               340                 345                 350
   Leu Glu Ala Gln Lys Leu Phe Val Asp Asn Lys Ile Leu Phe Ala Pro
           355                 360                 365
   Ala Lys Ala Ala Asn Ala Gly Gly Val Ala Thr Ser Ala Leu Glu Met
       370                 375                 380
   Ser Gln Asn Ser Leu Arg Met Ser Trp Thr Phe Glu Glu Val Asp Ala
   385                 390                 395                 400
   Lys Leu Lys Asp Ile Met Lys Asn Ile Tyr Tyr Asn Ser Arg Asn Ala
                   405                 410                 415
   Ala Ser Glu Tyr Gly His Asp Gly Asn Leu Ile Val Gly Ala Asn Ile
               420                 425                 430
   Ala Gly Phe Lys Lys Val Ala Asp Ala Met Leu Asp His Gly Ile Ile
           435                 440                 445
<210> 3
   <211> 421
   <212> PRT
   <213> Clostridium botulinum
<400> 3
   Met Ala Lys Glu Asn Leu Asn Pro Phe Glu Asn Ala Gln Lys Gln Val
   1               5                   10                  15
   Lys Thr Ala Cys Asp Lys Leu Gly Met Glu Pro Ala Val Tyr Glu Leu
               20                  25                  30
   Leu Lys Glu Pro Gln Arg Val Ile Glu Val Ser Ile Pro Val Lys Met
           35                  40                  45
   Asp Asp Gly Ser Val Lys Val Phe Lys Gly Tyr Arg Ser Gln His Asn
       50                  55                  60
   Asp Ala Val Gly Pro Thr Lys Gly Gly Val Arg Phe His Pro Asn Val
   65                  70                  75                  80
   Ser Leu Asp Glu Val Lys Ala Leu Ser Ile Trp Met Thr Phe Lys Cys
                   85                  90                  95
   Ser Val Thr Gly Ile Pro Tyr Gly Gly Gly Lys Gly Gly Ile Ile Val
               100                 105                 110
   Asp Pro Lys Thr Leu Ser Lys Gly Glu Leu Glu Arg Leu Ser Arg Gly
           115                 120                 125
   Tyr Ile Asp Gly Ile His Lys Leu Ile Gly Glu Lys Val Asp Val Pro
       130                 135                 140
   Ala Pro Asp Val Asn Thr Asn Gly Gln Ile Met Ala Trp Met Val Asp
   145                 150                 155                 160
   Glu Tyr Asn Lys Leu Val Gly Arg Ser Ala Ile Gly Val Ile Thr Gly
                   165                 170                 175
   Lys Pro Val Glu Phe Gly Gly Ser Leu Gly Arg Asn Ala Ala Thr Gly
               180                 185                 190
   Phe Gly Val Ala Val Thr Ala Arg Glu Ala Ala Ala Lys Leu Gly Ile
           195                 200                 205
   Asp Met Lys Lys Ala Lys Leu Ala Ile Gln Gly Ile Gly Asn Val Gly
       210                 215                 220
   Ser His Thr Val Leu Asn Cys Glu Lys Leu Gly Gly Thr Val Val Ala
   225                 230                 235                 240
   Leu Ala Glu Trp Cys Lys Glu Glu Gly Thr Tyr Ala Ile Tyr Asn Glu
                   245                 250                 255
   Asn Gly Leu Asp Gly Lys Ala Met Ile Glu Tyr Val Lys Glu Asn Gly
               260                 265                 270
   Asn Leu Leu Gly Tyr Pro Gly Ala Lys Lys Ile Ser Leu Asp Glu Phe
           275                 280                 285
   Trp Ala Leu Asn Val Asp Ile Leu Ile Pro Ala Ala Leu Glu Asn Ala
       290                 295                 300
   Ile Thr His Glu Asn Ala Ser Ser Ile Asn Ala Lys Leu Val Cys Glu
   305                 310                 315                 320
   Ala Ala Asn Gly Pro Ile Thr Pro Asp Ala Asp Ala Ile Leu Lys Glu
                   325                 330                 335
   Lys Gly Ile Thr Val Thr Pro Asp Ile Leu Thr Asn Ala Gly Gly Val
               340                 345                 350
   Thr Val Ser Tyr Phe Glu Trp Val Gln Asn Leu Tyr Gly Tyr Tyr Trp
           355                 360                 365
   Thr Glu Ala Glu Val Glu Ala Lys Glu Glu Glu Ala Met Val Lys Ala
       370                 375                 380
   Phe Glu Ser Ile Trp Ala Ile Lys Glu Glu Tyr Ser Val Thr Met Arg
   385                 390                 395                 400
   Glu Ala Ala Tyr Met His Ser Ile Lys Lys Val Ala Gly Ala Met Lys
                   405                 410                 415
   Leu Arg Gly Trp Tyr
               420